(19) Europäisches Patentamt
European Patent Office
Office européen des brevets

(11) EP 1 541 152 A1

(12) **EUROPÄISCHE PATENTANMELDUNG**

(43) Veröffentlichungstag:
**15.06.2005 Patentblatt 2005/24**

(51) Int Cl.[7]: **A61K 31/575**, A61K 31/12, A61K 35/78, A61P 17/00

(21) Anmeldenummer: **04027648.7**

(22) Anmeldetag: **22.11.2004**

(84) Benannte Vertragsstaaten:
**AT BE BG CH CY CZ DE DK EE ES FI FR GB GR HU IE IS IT LI LU MC NL PL PT RO SE SI SK TR**
Benannte Erstreckungsstaaten:
**AL HR LT LV MK YU**

(30) Priorität: **02.12.2003 DE 10356187**

(71) Anmelder: **Beiersdorf AG**
**20253 Hamburg (DE)**

(72) Erfinder:
- **Mummert, Christopher, Dr.**
  **29553 Bienenbüttel (DE)**
- **Eckert, Julia**
  **20257 Hamburg (DE)**
- **Raschke, Thomas, Dr.**
  **25421 Pinneberg (DE)**
- **Kruse, Inge**
  **20249 Hamburg (DE)**

(54) **Wirkstoffkombinationen aus Phytosterolen und/oder Cholesterin und Licochalcon A oder einem wässrigen Extrakt aus Radix Glycyrrhizae inflatae, enthaltend Licochalcon A**

(57) Wirkstoffkombinationen aus Phytosterolen und/oder Cholesterin und Licochalcon A oder einem wäßrigen Extrakt aus *Radix Glycyrrhizae inflatae,* enthaltend Licochalcon A, und deren Verwendung in Dermatologie.

EP 1 541 152 A1

## Beschreibung

Die vorliegende Erfindung betrifft kosmetische bzw. dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Bei alter Haut beispielsweise erfolgt die regenerative Erneuerung verlangsamt, wobei insbesondere das Wasserbindungsvermögen der Hornschicht nachläßt. Sie wird deshalb inflexibel, trocken und rissig ("physiologisch" trokkene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen.

Bei pathologisch trockener und empfindlicher Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Die Barrierewirkung der Haut kann über die Bestimmung des transepidermalen Wasserverlustes (TEWL - transepidermal water loss) quantifiziert werden. Dabei handelt es sich um die Abdunstung von Wasser aus dem Körperinneren ohne Einbeziehung des Wasserverlustes beim Schwitzen. Die Bestimmung des TEWL-Wertes hat sich als außerordentlich informativ erwiesen und kann zur Diagnose rissiger oder schrundiger Haut, zur Bestimmung der Verträglichkeit chemisch verschiedenartig aufgebauter Tenside und dergleichen mehr herangezogen werden.

Für die Schönheit und Gepflegtheit der Haut ist der Wasseranteil in der obersten Hautschicht von größter Bedeutung. Man kann ihn in einem begrenzten Umfang durch Einbringen von Feuchtigkeitsregulatoren günstig beeinflussen.

Anionische Tenside, welche im allgemeinen Bestandteile von Reinigungszubereitungen sind, können den pH-Wert in der Hornschicht langanhaltend erhöhen, was regenerative Prozesse, die der Wiederherstellung und Erneuerung der Barrierefunktion der Haut dienen, stark behindert. In diesem Fall stellt sich in der Hornschicht zwischen Regeneration und dem Verlust essentieller Substanzen durch regelmäßige Extraktion ein neuer, häufig sehr ungünstiger Gleichgewichtszustand ein, der das äußere Erscheinungsbild der Haut und die physiologische Funktionsweise der Hornschicht entscheidend beeinträchtigt.

Schon bei einem einfachen Wasserbade ohne Zusatz von Tensiden kommt es zunächst zu einer Quellung der Hornschicht d er H aut, w obei d er G rad d ieser Q uellung b eispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab- bzw. ausgewaschen. Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze noch verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch u mweltbedingte A bnutzungsschäden b zw. Irritationen, L ichtschäden, Altershaut usw., ist der Schutzmechanismus der Hautoberfläche gestört. Unter Umständen ist er dann aus eigener Kraft nicht mehr imstande, seine Aufgabe zu erfüllen und muss durch externe Maßnahmen regeneriert werden.

Darüber hinaus ist bekannt, daß Lipidzusammensetzung und -menge der Hornschicht der pathologisch veränderten, trockenen und der trockenen, jedoch nicht erkrankten Haut jüngerer und älterer Menschen vom Normalzustand abweicht, der in der gesunden, normal hydrierten Haut einer gleichalten Altersgruppe vorgefunden wird. Dabei stellen die Veränderungen im Lipidmuster der sehr trockenen, nicht-ekzematösen H aut von P atienten m it a topischem Ekzem einen Extremfall für die Abweichungen dar, die in der trockenen Haut hautgesunder Menschen vorgefunden werden.

Diese Abweichungen betreffen dabei ganz besonders die Ceramide, die in ihrer Menge stark reduziert und zusätzlich anders zusammengesetzt sind. Auffallend ist dabei in besonderer Weise das Defizit an den Ceramiden 1 und 3, wobei insbesondere für das Ceramid 1 bekannt ist, daß es in besonderer Weise die Ordnung der Lipide in den Interzellularmembransystemen steigert.

Nachteilige Veränderungen in den Lipidmembranen der vorab geschilderten Art beruhen möglicherweise auf fehlgesteuerter Lipidbiosynthese und erhöhen ebenfalls im Endeffekt den transepidermalen Wasserverlust. Eine langanhaltende Barriereschwäche wiederum macht die an sich gesunde Haut empfindlicher und kann im Einzellfalle zum Entstehen ekzematöser Vorgänge in der kranken Haut beitragen.

Die Wirkung von Salben und Cremes auf Barrierefunktion und Hydratation der Hornschicht besteht in der Regel nicht in einer Wiederherstellung bzw. Stärkung der physikalisch-chemischen Eigenschaften der Lamellen aus Interzellularlipiden. Ein wesentlicher Teileffekt beruht auf der bloßen Abdeckung der behandelten Hautbezirke und dem daraus resultierenden Wasserstau in der darunterliegenden Hornschicht. Coapplizierte hygroskopische Substanzen binden das Wasser, so dass es zu einer messbaren Zunahme des Wassergehaltes in der Hornschicht kommt. Diese rein physikalische Barriere kann jedoch relativ leicht wieder entfernt werden. Nach dem Absetzen des Produktes kehrt die Haut dann sehr schnell wieder den Zustand vor Behandlungsbeginn zurück. Darüber hinaus kann die Hautpflegewirkung bei regelmäßiger Behandlung nachlassen, so dass schließlich sogar während der Behandlung der Status quo wieder erreicht wird. Bei bestimmten Produkten verschlechtert sich der Zustand der Haut nach Absetzen unter Umständen vorübergehend. Eine nachhaltige Produktwirkung wird in der Regel also nicht oder nur in einem eingeschränkten Maße erreicht.

Um die defizitäre Haut bei ihrer natürlichen Regeneration zu unterstützen und ihre physiologische Funktion zu stärken, werden topischen Präparaten in neuerer Zeit zunehmend Interzellularlipidmischungen zugesetzt, die von der Haut zum Wiederaufbau der natürlichen Barriere verwendet werden sollen. Allerdings handelt es sich bei diesen Lipiden, insbesondere aber den Ceramiden, um sehr teure Rohstoffe. Zudem ist ihre Wirkung meist sehr viel geringer als erhofft.

Ziel der vorliegenden Erfindung war es somit, Wege zu finden, die Nachteile des Standes der Technik zu vermeiden. Insbesondere sollte die Wirkung der Hautpflegeprodukte physiologisch, schnell und nachhaltig sein.

Unter Hautpflege im Sinne der vorliegenden Erfindung ist in erster Linie zu verstehen, daß die natürliche Funktion der Haut als Barriere gegen Umwelteinflüsse (z. B. Schmutz, Chemikalien, Mikroorganismen) und gegen den Verlust von körpereigenen Stoffen (z. B. Wasser, Lipide, Elektrolyte) gestärkt oder wiederhergestellt wird.

Produkte zur Pflege, Behandlung und Reinigung trockener und strapazierter Haut sind an sich bekannt. Allerdings ist ihr Beitrag zur Regeneration einer physiologisch intakten, hydratisierten und glatten Hornschicht umfangmäßig und zeitlich begrenzt.

Die Wirkung von Salben und Cremes auf die Barrierefunktion und die Hydratation der Hornschicht beruht im wesentlichen auf der Abdeckung (Okklusion) der behandelten Hautbezirke. Die Salbe oder Creme stellt sozusagen eine (zweite) künstliche Barriere dar, die den Wasserverlust der Haut Verhindern soll. Entsprechend leicht kann diese physikalische Barriere - beispielsweise mit Reinigungsmitteln - wieder entfernt werden, wodurch der ursprüngliche, beeinträchtigte Zustand wieder erreicht wird. Darüber hinaus kann die Hautpflegewirkung bei regelmäßiger Behandlung nachlassen. Nach dem Absetzen der Produktanwendung kehrt die Haut sehr schnell wieder in den Zustand vor Behandlungsbeginn zurück. Bei bestimmten Produkten verschlechtert sich der Zustand der Haut unter Umständen sogar vorübergehend. Eine nachhaltige Produktwirkung wird in der Regel also nicht oder nur in einem eingeschränkten Maße erreicht.

Die Wirkung einiger pharmazeutischer Zubereitungen auf die Barrierefunktion der Haut besteht sogar in einer selektiven Barriereschädigung, die ermöglichen soll, daß Wirkstoffe in bzw. durch die Haut in den Körper eindringen können. Ein gestörtes Erscheinungsbild der Haut wird dabei als Nebenwirkung teilweise billigend in Kauf genommen.

Die Wirkung von pflegenden Reinigungsprodukten besteht im wesentlichen in einer effizienten Rückfettung mit Sebumlipid-ähnlichen Substanzen. Durch die gleichzeitige Verminderung des Tensidgehalts solcher Zubereitungen lässt sich der Schaden an der Hornschichtbarriere weiter begrenzen.

Dem Stand der Technik mangelt es allerdings an Zubereitungen, welche die Barrierefunktion und die Hydratation der Hornschicht positiv beeinflussen und die physikalisch-chemischen Eigenschaften der Hornschicht und insbesondere der Lamellen aus Interzellularlipiden stärken bzw. sogar wiederherstellen.

Aufgabe der vorliegenden Erfindung war es also, die Nachteile des Standes der Technik zu beseitigen. Insbesondere sollten hautpflegende Zubereitungen und Zubereitungen zur Reinigung der Haut zur Verfügung gestellt werden, welche die Barriereeigenschaften der Haut erhalten oder wiederherstellen, zumal dann, wenn die natürliche Regeneration der Haut nicht ausreicht. Sie sollen ferner zur Behandlung und Prophylaxe von Folgeschäden der Hautaustrocknung, beispielsweise Fissuren oder inflammatorischen oder allergischen Prozessen oder auch der Neurodermitis, geeignet sein. Aufgabe der vorliegenden Erfindung war es auch, stabile hautpflegende kosmetische und/oder dermatologische Mittel zur Verfügung zu stellen, welche die Haut vor Umwelteinflüssen wie Sonne und Wind schützen. Insbesondere sollte die Wirkung der Zubereitungen physiologisch, schnell und nachhaltig sein.

Darüber hinaus betrifft die Erfindung Zubereitungen mit extrem niedrigem sogenanntem "Stinging Potential". Bei Menschen mit sensibler, empfindlicher oder verletzlicher Haut kann ein mit "Stinging" (<engl.> "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

Als neurosensorisches Phänomen ist der Juckreiz bei atopischer Haut anzusehen, sowie Juckreiz bei Hauterkrankungen.

"Stinging"-Phänomene können als kosmetisch zu behandelnde Störungen angesehen werden. Starker Juckreiz dagegen, insbesondere bei Atopie auftretendes starkes Hautjucken, kann auch als schwerwiegendere dermatologische Störung bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen z.B. Massage, Tensideinwirkung, Wettereinfluss wie Sonne, Kälte, Trokkenheit, aber auch feuchte Wärme, Wärmestrahlung und UV-Strahlung, z.B. der Sonne, hervorgerufen werden.

In "Journal of the Society of Cosmetic Chemists" 28, S.197 - 209 (Mai 1977) beschreiben P.J.Frosch und A. M.Kligman eine Methode zur Abschätzung des "Stinging-Potentials" topisch verabreichter Substanzen. Als positive Substanzen werden hier z.B. Milchsäure und Brenztraubensäure eingesetzt. Bei Messung nach dieser Methode wurden aber auch Aminosäuren, insbesondere Glycin, als neurosensorisch aktiv ermittelt (solche Substanzen werden "Stinger" genannt).

Nach bisherigen Erkenntnissen tritt eine derartige Empfindlichkeit gegenüber ganz bestimmten Substanzen individuell unterschiedlich auf. Dies bedeutet, eine Person, die bei Kontakt mit einer Substanz "Stingingeffekte" erlebt, wird sie mit hoher Wahrscheinlichkeit bei jedem weiteren Kontakt wiederholt erleben. Der Kontakt mit anderen "Stingern" kann aber ebensogut ohne jede Reaktion verlaufen.

Das Problem "sensible Haut" betrifft eine wachsende Anzahl Erwachsenen und Kindern. Mit Sensibler Haut bezeichnet man eine Kombination verschiedener Symptome, wie hyperreaktive und intolerante Haut. Aber auch atopische Haut kann darunter s ubsummiert werden. Diese H autzustände w erden von d en B etroffenen o ftmals, n icht g anz k orrekt, als "allergische" Haut bezeichnet. Obwohl eine allergische Erkrankung zu Symptomen der sensiblen Haut führen kann, ist die Erscheinung "sensible Haut" nicht auf Allergiker beschränkt

Viele mehr oder weniger empfindliche Personen haben auch bei Verwendung mancher desodorierenden oder antitranspirierend wirkenden Zubereitungen unter erythematösen Hauterscheinungen zu leiden.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Es war also die Aufgabe der vorliegenden Erfindung, den Nachteilen des Standes der Technik abzuhelfen.

Insbesondere sollten Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, insbesondere Dermatosen, aber auch des Erscheinungsbildes des "Stingings" zur Verfügung gestellt werden.

Ferner sollten solche Wirkstoffe, bzw. Zubereitungen, solche Wirkstoffe enthaltend, zur Verfügung g estellt w erden, welche zur Immunstimulation der Haut, dabei vorteilhaft auch zur Immunstimulation im Sinne der die Wundheilung fördernden Wirkung, verwendet werden können.

In einer weiteren bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der unerwünschten Pigmentierung, beispielsweise lokale Hyper- und Fehlpigmentierungen (beispielsweise Leberflecken, Sommersprossen), aber auch zur rein kosmetischen Aufhellung größerer, dem individuellen Hauttyp an sich durchaus angemessen pigmentierter Hautflächen.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende pigmentbildende Zellen vorzufinden sind. Melanozyten enthalten als charakteristische Zellorganellen Melanosomen, die bei Anregung durch UV-Strahlung verstärkt Melanin bilden. Dieses wird in die Keratinozyten transportiert und ruft eine mehr oder weniger ausgeprägte bräunliche oder braune Hautfarbe hervor.

Melanin wird als Endstufe eines oxidativen Prozesses gebildet, in welchem Tyrosin unter Mitwirkung des Enzymes Tyrosinase über 3,4-Dihydroxyphenylalanin (Dopa), Dopa-Chinon, Leucodopachrom, Dopachrom, 5,6-Dihydroxyindol und Indol-5,6-chinon schließlich in Melanin umgewandelt wird.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides*), genetische Disposition, Fehlpigmentierung der Haut bei der Wundheilung bzw. -vernarbung oder der Hautalterung (z.B. *Lentigines seniles*).

Es sind Wirkstoffe und Zubereitungen bekannt, welche der Hautpigmentierung e ntgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Auch die Inhibierung der Tyrosinase mit Substanzen wie Kojisäure, Ascorbinsäure und Azelainsäure sowie deren Derivaten ist geläufig, hat aber kosmetische und dermatologische Nachteile.

Auch diesen Übelständen abzuhelfen, war Aufgabe der vorliegenden Erfindung.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:

a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:

d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/ oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vorschädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen

können. Es ist erwiesen, daß UVA-Strahlung zu einer Schädigung der elastischen und kollagenen Fasern des Bindegewebes führt, was die Haut vorzeitig altern lässt, und dass sie als Ursache zahlreicher phototoxischer und photoallergischer Reaktionen zu sehen ist. Der schädigende Einfluss der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxylradikale, Singulettsauerstoff. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/ Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Erfindungsgemäß werden die Übelstände des Standes der Technik beseitigt durch Wirkstoffkombinationen aus

(a) einem oder mehreren Vertreter der Gruppe bestehend aus Cholesterin und/oder Phytosterolen und
(b) Licochalcon A oder einem wäßrigen Extrakt aus Radix Glycyrrhizae inflatae, enthaltend Licochalcon A

bzw. kosmetische oder dermatologische Zubereitungen, solche Wirkstofffkombinationen enthaltend.

Zubereitungen gemäß der Erfindung bzw. kosmetische oder dermatologische Zubereitungen, sind in jeglicher Hinsicht überaus befriedigende Präparate. Es war für den Fachmann nicht vorauszusehen, daß die Zubereitungen gemäß der Erfindung

- besser die Barriereeigenschaften der Haut erhalten oder wiederherstellen,
- besser der Hautaustrocknung entgegenwirken,
- besser gegen Pigmentstörungen wirken,
- besser gegen entzündliche Hautzustände wirken

- besser gegen die Hautalterung wirken und
- die Haut besser vor Umwelteinflüssen schützen

als die Zubereitungen des Standes der Technik.

Die Verwendung erfindungsgemäßer Wirkstofffkombinationen bzw. kosmetischer oder topischer dermatologischer Zubereitungen mit einem wirksamen Gehalt an erfindungsgemäßen Wirkstofffkombinationen bietet in überraschender Weise eine wirksame Behandlung, aber auch eine Prophylaxe

- von defizitären, sensitiven oder hypoaktiven Hautzuständen oder defizitären, sensitiven oder hypoaktiven Zustände von Hautanhangsgebilden
- von Erscheinungen vorzeitiger Alterung der Haut (z.B. Falten, Altersflecken, Teleangiektasien) und/oder der Hautanhangsgebilde,
- von umweltbedingten (Rauchen, Smog, reaktive Sauerstoffspecies, freie Radikale) und insbesondere lichtbedingten negativen Veränderungen der Haut und der Hautanhangsgebilde.
- von lichtbedingten Hautschäden
- von Pigmentierungsstörungen,
- von empfindlicher, gereizter und juckender Haut,
- von trockenen Hautzuständen und Hornschichtbarrierestörungen,
- von Haarausfall und für verbessertes Haarwachstum
- von entzündlichen Hautzuständen sowie atopischem Ekzem, seborrhoischem Ekzem, polymorpher Lichtdermatose, Psoriasis, Vitiligo

möglich. Der erfindungsgemäße Wirkstoffes bzw. kosmetischer oder topischer dermatologischer Zubereitungen mit einem wirksamen Gehalt an erfindungsgemäßem Wirkstoff dient aber auch in überraschender Weise

- zur Beruhigung von empfindlicher oder gereizter Haut
- zur Stimulation der Kollagen-, Hyaluronsäure-, Elastinsynthese
- zur Stimulation der Ceramidsynthese der Haut
- zur Stimulation der intrazellulären DNA-Synthese, insbesondere bei defizitären oder hypoaktiven Hautzuständen.
- zur Steigerung der Zellerneuerung und Regeneration der Haut
- zur Steigerung der hauteigenen Schutz- und Reparaturmechanismen (beispielsweise für dysfunktionelle Enzyme, DNA, Lipide, Proteine)
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern.

Vorzugsweise liegen in den erfindungsgemäßen Wirkstoffkombinationen das oder die Phytosterole oder Cholesterin und Licochalcon A in Verhältnissen von 1000 : 1 bis 1 : 100, bevorzugt 100 : 1 bis 1 : 25, besonders bevorzugt 5 : 1 bis 1 : 5 vor.

Phytosterole, auch Phytosterine genannt, sind Sterole, die in Pflanzen und Hefen gefunden werden. Letztere werden auch als Mykosterine bezeichnet. Im Gegensatze zu tierischen Sterinen sind Phytosterole in C-24-Position mit einem $C_1$ - oder $C_2$ - Rest substituiert und besitzen in C-22-Position meist eine Doppelbindung.

Beispiele für Phytosterole sind:

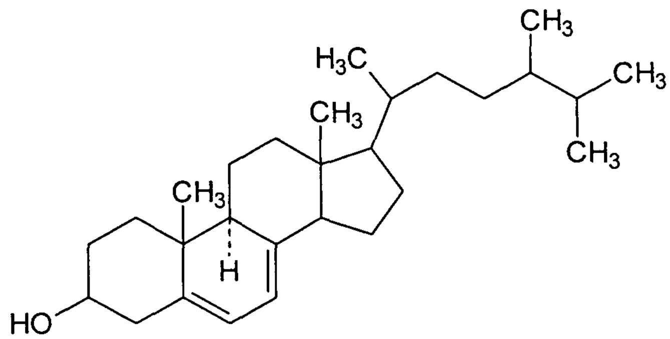

(Ergosterol),

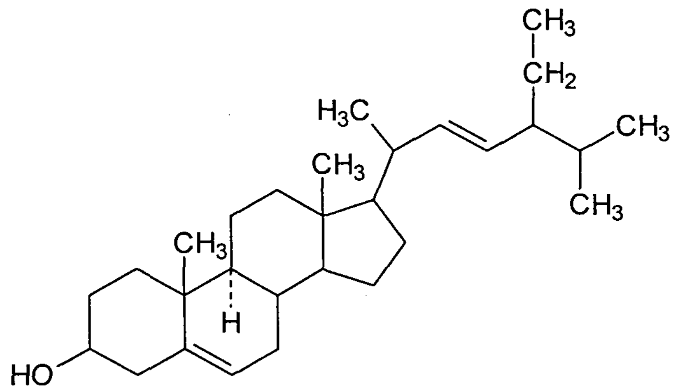

(Stigmasterol),

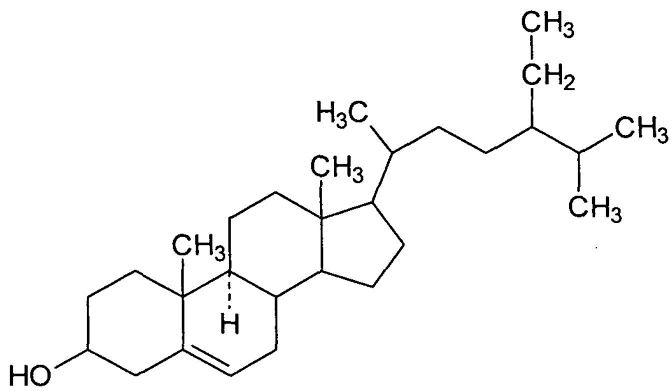

(Sitosterol),

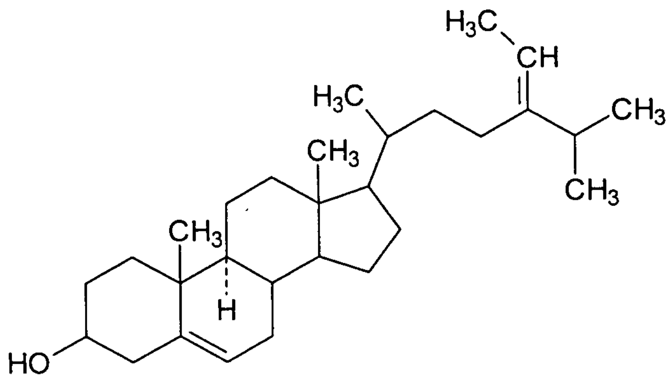

(Fucosterol),

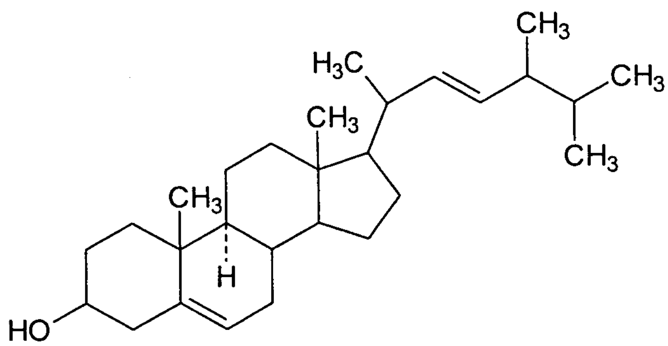

(Brassicasterol),

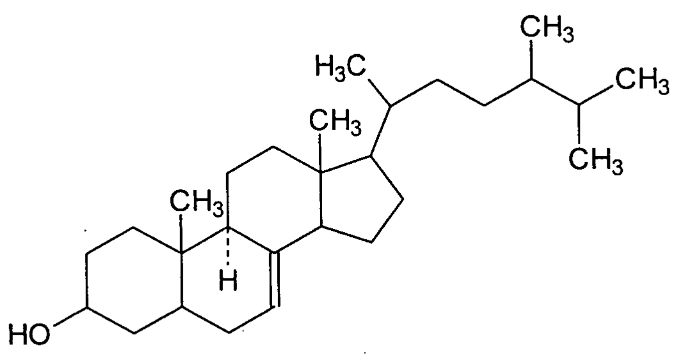

(Fungisterol),

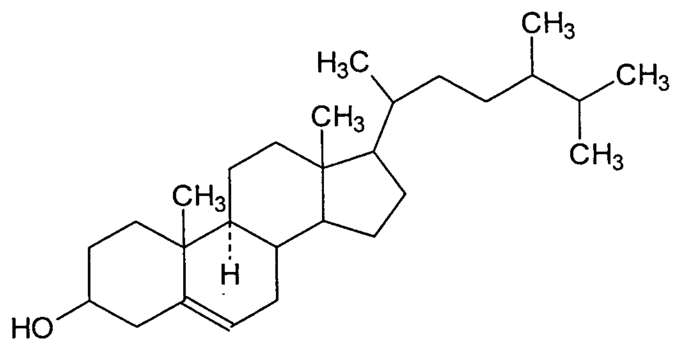

(Campesterol).

Alle erfindungsgemäß zu verwendenden Phytosterole besitzen eine mehr oder weniger große Anzahl an optischen Isomeren, welche hier im einzelnen nicht aufgeführt werden sollen, welche sich aber als vorteilhaft erwiesen haben, sofern ihre kosmetische Unbedenklichkeit außer Frage steht.

Bevorzugte Phytosterole sind Sitosterol, Campesterol, Stigmasterol.

Ein bekannter kosmetischer Rohstoff ist das Cholesterin. Es zeichnet sich durch folgende Struktur aus:

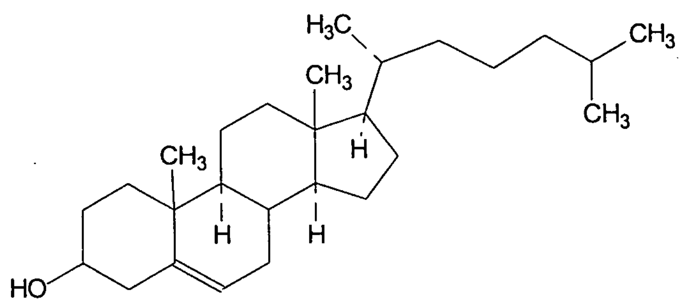

Vorteilhaft enthalten erfindungsgemäße Zubereitungen 0,00001 - 5 Gew.-% eines oder mehrerer Phytosterole, oder Cholesterin, bevorzugt 0,0001 - 2 Gew.-%, insbesondere 0,01 - 1 Gew.-%, jeweils bezogen auf das Gesamtgewicht der Zubereitungen.

Die Pflanzenart *Glycyrrhiza inflata* gehört wie das in Europa offizinelle Süßholz *Glycyrrhiza glabra* der Gattung *Glycyrrhiza* an, die zur Pflanzenfamilie der Fabaceae (Erbsengewächse) gehört. Die Droge *Radix Glycyrrhizae inflatae,* d.h. die Wurzel der Pflanze, ist beispielsweise in der fernöstlichen Medizin gebräuchlich. Die Verwendung der Droge als Entzündungshemmer ist ebenfalls bekannt.

Ein Bestandteil des wäßrigen Auszugs aus Radix Glycyrrhizae inflatae ist das Licochalcon A, welches sich durch die folgende Strukturformel auszeichnet:

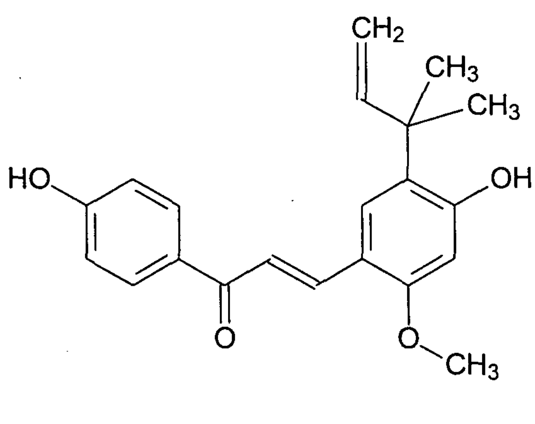

Es wird angenommen, daß diese Substanz, möglicherweise in Synergie mit den übrigen Bestandteilen des Extraktes, Anteil an der erfindungsgemäßen Wirkung besitzt.

Vorteilhaft ist erfindungsgemäß insbesondere, wenn die Zubereitungen 0,0001 bis 5 Gew.-%, insbesondere 0,001 bis 1 Gew.-%, ganz besonders 0,005 bis 0,15 Gew.-% Licochalcon A enthalten, bezogen auf das Gesamtgewicht der Zubereitung.

Vorteilhaft ist erfindungsgemäß ferner insbesondere, wenn die Zubereitungen 0,001 bis 20 Gew.-%, insbesondere 0,05 bis 10 Gew.-%, ganz besonders 0,01 bis 5 Gew.-% an einem oder mehreren Polyolen enthalten, bezogen auf das Gesamtgewicht der Zubereitung.

Verwendungsgemäße Polyole sind Glycerin, Butylenglycol, Dipropylenglycol, Propylenglycol, Pentandiol, Hexandiol.

Vorteilhaft ist erfindungsgemäß ferner wenn die Zubereitungen Licocalchon als Bestandteil von pflanzlichen Extracten, insbesondere von *Radix Glycyrrhizae inflatae,* enthalten.

Erfindungsgemäß ist ferner vorteilhaft, wenn Licochalcon in Form eines wäßrigen Auszugs vorliegt, in welchem

- Licochalcon A
- Wasser
- gegebenenfalls ein oder mehrere Polyole

vorliegen.

Es ist erfindungsgemäß vorteilhaft, wenn die kosmetischen oder dermatologischen Zubereitungen 0,0001 bis 10 Gew.-%, insbesondere 0,005 bis 5 Gew.-%, ganz besonders 0,01 bis 1 Gew.-% an einem wäßrigen Extrakt aus *Radix Glycyrrhizae inflatae* enthalten, bezogen auf das Gesamtgewicht der Zubereitung.

Es ist erfindungsgemäß vorteilhaft, wenn die kosmetischen oder dermatologischen Zubereitungen 0,001 bis 20 Gew.-%, insbesondere 0,05 bis 10 Gew.-%, ganz besonders 0,01 bis 5 Gew.-% an einem oder mehreren Polyolen enthalten, bezogen auf das Gesamtgewicht der Zubereitung.

Insbesondere ist vorteilhaft, als Polyol das Butylenglycol zu wählen.

Ganz besonders vorteilhaft ist es, von einem Extrakt auszugehen, der unter der Bezeichnung Polyol Soluble Licorice Extract P-U von der Firma Maruzen vertrieben wird.

Ferner ist es vorteilhaft, Licochalcone A in anderen Vehikelsystemen in einer Konzentration von 0,0001 bis 5 Gew.-%, insbesondere 0,001 bis 1 Gew.-%, ganz besonders 0,005 - 0,05 Gew.-% zu verwenden.

Erfindungsgemäß ist demnach auch die Verwendung erfindungsgemäßer Wirkstofffkombinationen zur Prophylaxe und Behandlung von entzündlichen Hautzuständen - auch dem a-topischen Ekzem - und/oder zum Hautschutz bei empfindlich determinierter trockener Haut.

Erfindungsgemäß ist demnach auch die Verwendung erfindungsgemäßer Wirkstofffkombinationen zur Herstellung von kosmetischen oder dermatologischen Zubereitungen zur Herstellung von kosmetischen oder dermatologischen Zubereitungen zur Behandlung und/oder Prophylaxe von Pigmentierungsstörungen.

Erfindungsgemäß ist demnach auch die Verwendung erfindungsgemäßer Wirkstofffkombinationen zur Herstellung von kosmetischen oder dermatologischen Zubereitungen zur Behandlung und/oder Prophylaxe der Symptome der intrinsischen und/oder extrinsischen Hautalterung sowie zur Behandlung und Prophylaxe der schädlichen Auswirkungen ultravioletter Strahlung auf die Haut

Erfindungsgemäß ist demnach auch die Verwendung erfindungsgemäßer Wirkstofffkombinationen zur Herstellung von kosmetischen oder dermatologischen Zubereitungen zur Steigerung der Ceramidbiosynthese.

Erfindungsgemäß ist demnach auch die Verwendung erfindungsgemäßer Wirkstofffkombinationen zur Herstellung von kosmetischen oder dermatologischen Zubereitungen zur Stärkung der Barrierefunktion der Haut.

Bevorzugt enthalten kosmetische oder dermatologische Zubereitungen gemäß der Erfindung 0,0001 - 10 Gew.-%, besonders bevorzugt 0,001 - 1 Gew.-%, an erfindungsgemäßen Wirkstofffkombinationen, bezogen auf die Gesamtzusammensetzung der Zubereitungen.

Es ist erfindungsgemäß insbesondere äußerst vorteilhaft, erfindungsgemäße Wirkstofffkombinationen bzw. kosmetische oder topische dermatologische Zubereitungen mit einem wirksamen Gehalt an erfindungsgemäßen Wirkstofffkombinationen zur kosmetischen oder dermatologischen Behandlung oder Prophylaxe unerwünschter Hautzustände zu verwenden.

Erfindungsgemäß können Zubereitungen welche erfindungsgemäße Wirkstofffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nu-kleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Vorteilhaft kann der erfindungsgemäß, verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Creme, einer Lotion, eines Schaums, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Die erfindungsgemäßen kosmetischen Zubereitungen können daher kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Ebenfalls geeignete UV-A Filtersubstanzen sind Hydroxybenzophenone. Diese zeichnen sich durch die fol-

gende Strukturformel aus:

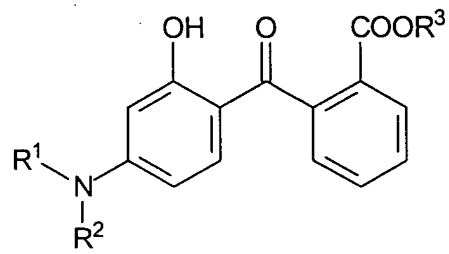

worin

- $R^1$ und $R^2$ unabhängig voneinander Wasserstoff, $C_1$-$C_{20}$-Alkyl, $C_3$-$C_{10}$-Cycloalkyl oder $C_3$-$C_{10}$-Cycloalkenyl bedeuten, wobei die Substituenten $R^1$ und $R^2$ gemeinsam mit dem Stickstoffatom, an das sie gebunden sind, einen 5- oder 6-Ring bilden können und

- $R^3$ einen $C_1$-$C_{20}$-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet:

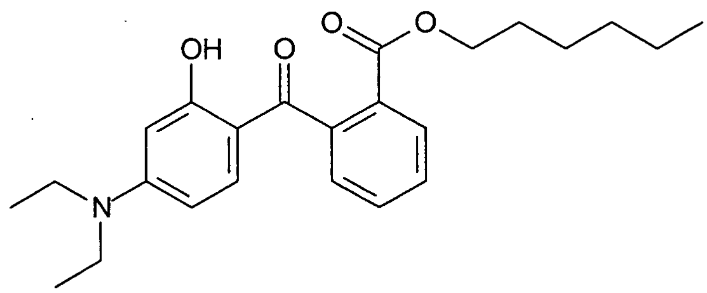

und unter dem Uvinul A Plus bei der Fa. BASF erhältlich ist.
Die Gesamtmenge an einem oder mehreren Hydroxybenzophenonen in den fertigen kosmetischen oder dermatologischen Zubereitungen wird vorteilhaft aus dem Bereich 0,01 Gew.-% bis 20 Gew.-%, vorzugsweise von 0,1 bis 10 Gew.-% gewählt, jeweils bezogen auf das Gesamtgewicht der Zubereitungen.
Vorteilhafte weitere UV-Filtersubstanzen im Sinne der vorliegenden Erfindung sind sulfonierte, wasserlösliche UV-Filter, wie z. B.:

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;

- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;

- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfatoverbindungen, insbesondere das entsprechende Natrium-, Kalium- oder Triethanolammonium-Salz), das auch als

Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;

- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.
Vorteilhafte Breitbandfilter oder UV-B-Filtersubstanzen sind beispielsweise Triazinderivate, wie z. B.

- 2,4-Bis-{[4-(2-Ethyl-hexyloxy)-2-hydroxy]-phenyl}-6-(4-methoxyphenyl)-1,3,5-triazin (IN-CI: Aniso Triazin), welches unter der Handelsbezeichnung Tinosorb® S bei der CIBA-Chemikalien GmbH erhältlich ist;

- Diethylhexylbutylamidotriazon (INCI: Diethylhexylbutamidotriazone), welches unter der Handelsbezeichnung UVASORB HEB bei Sigma 3V erhältlich ist;

- 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVI-NUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist. Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.
Die weiteren UV-Filtersubstanzen können öllöslich oder wasserlöslich sein.
Vorteilhafte öllösliche UV-B- und/oder Breitband-Filtersubstanzen im Sinne der vorliegenden Erfindung sind z. B.:

- ■ 3-Benzylidencampher-Derivate, vorzugsweise 3-(4-Methylbenzyliden)campher, 3-Benzylidencampher;

- ■ 4-Aminobenzoesäure-Derivate, vorzugsweise 4-(Dimethylamino)-benzoesäure(2-ethylhexyl)ester, 4-(Dimethylamino)benzoesäureamylester;

- ■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon

- ■ sowie an Polymere gebundene UV-Filter.

- ■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Vorteilhafte wasserlösliche Filtersubstanzen sind z. B. Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.
Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.
Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-Aund/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder d as 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.
Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

$$n\ TiO_2 + m\ (RO)_3\ Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Erfindungsgemäße Zubereitungen können, zumal wenn kristalline oder mikrokristalline Festkörper, beispielsweise anorganische Mikropigmente in die erfindungsgemäßen Zubereitungen eingearbeitet werden sollen, auch anionische, nichtionische und/oder amphotere Tenside enthalten. Tenside sind amphiphile Stoffe, die organische, unpolare Substanzen in Wasser lösen können.

Bei den hydrophilen Anteilen eines Tensidmoleküls handelt es sich meist um polare funktionelle Gruppen, beispielweise $-COO^-$, $-OSO_3^{2-}$, $-SO_3^-$, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

$$RNH_2^+CH_2CH_2COOH\ X^- \qquad (\text{bei pH=2}) \qquad X^- = \text{beliebiges Anion, z.B. } Cl^-$$

$$RNH_2^+CH_2CH_2COO^- \qquad (\text{bei pH=7})$$

$$RNHCH_2CH_2COO^-\ B^+ \qquad (\text{bei pH=12}) \qquad B^+ = \text{beliebiges Kation, z.B. } Na^+$$

Typisch für nicht-ionische T enside s ind P olyether-Ketten. N icht-ionische T enside b ilden i n wäßrigem

Medium keine Ionen.

A. Anionische Tenside

Vorteilhaft zu verwendende anionische Tenside sind

Acylaminosäuren (und deren Salze), wie

1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. AcylLactylate, lauroyllactylat, Caproyllactylat
6. Alaninate

Carbonsäuren und Derivate, wie

1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
**[0129]** Sulfonsäuren und Salze, wie

1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium $C_{12-14}$ Olefinsulfonat, Natriumlauryl-sulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat

sowie
Schwefelsäureester, wie

1. Alkylethersulfat, beispielsweise Natrium-, Ammonium-, Magnesium-, MIPA-, TIPA-Laurethsulfat, Natriummy-rethsulfat und Natrium $C_{12-13}$ Parethsulfat,
2. Alkylsulfate, beispielsweise Natrium-, Ammonium- und TEA- Laurylsulfat.

B. Kationische Tenside

Vorteilhaft zu verwendende kationische Tenside sind

1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

C. Amphotere Tenside

Vorteilhaft zu verwendende amphotere Tenside sind

1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

D. Nicht-ionische Tenside

Vorteilhaft zu verwendende nicht-ionische Tenside sind

1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.
Die oberflächenaktive Substanz kann in einer Konzentration zwischen 1 und 95 Gew.-% in den erfindungsgemäßen Zubereitungen vorliegen, bezogen auf das Gesamtgewicht der Zubereitungen.
Die Lipidphase der erfindungsgemäßen kosmetischen oder dermatologischen Emulsionen kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/ oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.
Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Solche Silicone oder Siliconöle können als Monomere vorliegen, welche in der Regel durch Strukturelemente charakterisiert sind, wie folgt:

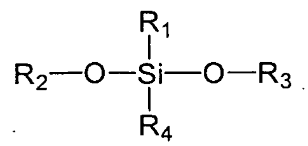

Als erfindungsgemäß vorteilhaft einzusetzenden linearen Silicone mit mehreren Siloxyleinheiten werden im allgemeinen durch Strukturelemente charakterisiert wie folgt:

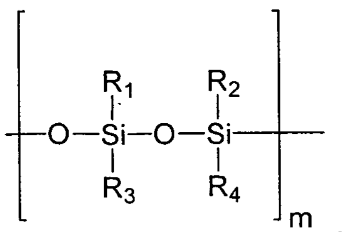

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende cyclische Silicone werden im allgemeinen durch Strukturelemente charakterisiert, wie folgt

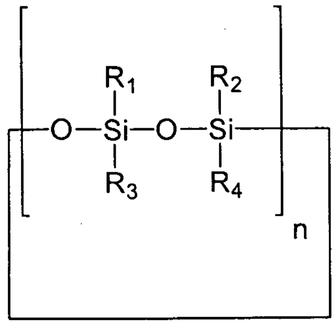

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Cyclomethicon (z.B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan), Cetyldimethicon, Behenoxydimethicon.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend b ezeichneten Verbindungen zu wählen, deren organische Seitenketten derivatisiert, beispielsweise polyethoxyliert und/oder polypropoxyliert sind. Dazu zählen beispielsweise Polysiloxanpolyalkyl-polyether-copolymere wie das Cetyl-Dimethicon-Copolyol, das (Cetyl-Dimethicon-Copolyol (und) Polyglyceryl-4-Isostearat (und) Hexyllaurat)

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate.

Erfindungsgemäße als Emulsionen vorliegenden Zubereitungen enthalten insbesondere vorteilhaft ein oder mehrere Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Erfindungsgemäße als Hydrogele vorliegenden Zubereitungen enthalten ein oder mehrere Hydrocolloide. Diese Hydrocolloide können vorteilhaft aus der vorgenannten Gruppe gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum@. Diese können als solche oder in modifizierter Form verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Goodrich (Carbopol 980, 981, 1382, 5984, 2984, EDT 2001 oder Pemulen TR2).

Unter den Polymeren befinden sich z.B. Polyacrylamide (Seppigel 305), Polyvinylalkohole, PVP, PVP / VA Copolymere, Polyglycole und Ammoniumpolyacryloyldimethyltaurate sowie Ammoniumacryloyldimethyltaurate/ Vinylpyrrolidoncopolymere.

Erfindungsgemäße als Emulsionen vorliegenden Zubereitungen enthalten einen oder mehrere Emulgatoren. Diese Emulgatoren können vorteilhaft gewählt werden aus der Gruppe der nichtionischen, anionischen, kationischen oder amphoteren Emulgatoren.

Unter den nichtionischen Emulgatoren befinden sich

a) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate)
b) ethoxylierte Fettalkohole und Fettsäuren
c) ethoxilierte Fettamine, Fettsäureamide, Fettsäurealkanolamide
d) Alkylphenolpolyglycolether (z.B. Triton X)

Unter den anionischen Emulgatoren befinden sich

a) Seifen (z. B. Natriumstearat)
b) Fettalkoholsulfate
c) Mono-, Di- und Trialkylphosphosäureester und deren Ethoxylate

Unter den kationischen Emulgatoren befinden sich

a) quaternäre Ammoniumverbindungen mit einem langkettigen aliphatischen Rest z.B. Distearyldimonium Chloride

Unter den amphoteren Emulgatoren befinden sich

a) Alkylamininoalkancarbonsäuren
b) Betaine, Sulfobetaine
c) Imidazolinderivate

Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:

- der Fettalkoholethoxylate
- der ethoxylierten Wollwachsalkohole,
- der Polyethylenglycolether der allgemeinen Formel $R\text{-}O\text{-}(\text{-}CH_2\text{-}CH_2\text{-}O\text{-})_n\text{-}R'$,
- der Fettsäureethoxylate der allgemeinen Formel

$$R\text{-}COO\text{-}(\text{-}CH_2\text{-}CH_2\text{-}O\text{-})_n\text{ -}H,$$

- der veretherten Fettsäureethoxylate der allgemeinen Formel

$$R\text{-}COO\text{-}(\text{-}CH_2\text{-}CH_2\text{-}O\text{-})_n\text{-}R',$$

- der veresterten Fettsäureethoxylate der allgemeinen Formel

$$R\text{-}COO\text{-}(\text{-}CH_2\text{-}CH_2\text{-}O\text{-})_n\text{ -}C(O)\text{-}R',$$

- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

$$R\text{-}O\text{-}(\text{-}CH_2\text{-}CH_2\text{-}O\text{-})_n\text{-}CH_2\text{-}COOH$$

nd n eine Zahl von 5 bis 30 darstellen,

- der Polyoxyethylensorbitolfettsäureester,
- der Alkylethersulfate der allgemeinen Formel $R\text{-}O\text{-}(\text{-}CH_2\text{-}CH_2\text{-}O\text{-})_n\text{-}SO_3\text{-}H$
- der Fettalkoholpropoxylate der allgemeinen Formel

$$R\text{-}O\text{-}(\text{-}CH_2\text{-}CH(CH_3)\text{-}O\text{-})_n\text{-}H,$$

- der Polypropylenglycolether der allgemeinen Formel

$$R\text{-}O\text{-}(\text{-}CH_2\text{-}CH(CH_3)\text{-}O\text{-})_n\text{-}R'$$

- der propoxylierten Wollwachsalkohole,
- der veretherten Fettsäurepropoxylate

$$R\text{-}COO\text{-}(\text{-}CH_2\text{-}CH(CH_3)\text{-}O\text{-})_n\text{-}R',$$

- der veresterten Fettsäurepropoxylate der allgemeinen Formel

$$R\text{-}COO\text{-}(\text{-}CH_2\text{-}CH(CH_3)\text{-}O\text{-})_n\text{-}C(O)\text{-}R',$$

- der Fettsäurepropoxylate der allgemeinen Formel

$$R\text{-}COO\text{-}(\text{-}CH_2\text{-}CH(CH_3)\text{-}O\text{-})_n\text{-}H,$$

- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

$$R\text{-}O\text{-}(\text{-}CH_2\text{-}CH(CH_3)O\text{-})_n\text{-}CH_2\text{-}COOH$$

- der Alkylethersulfate bzw. die diesen Sulfaten zugrundeliegenden Säuren der allgemeinen Formel $R\text{-}O\text{-}(\text{-}CH_2\text{-}CH(CH_3)\text{-}O\text{-})_n\text{-}SO_3\text{-}H$
- der Fettalkoholethoxylate/propoxylate der allgemeinen Formel

$$R\text{-}O\text{-}X_n\text{-}Y_m\text{-}H,$$

- der Polypropylenglycolether der allgemeinen Formel

$$R\text{-}O\text{-}X_n\text{-}Y_m\ R',$$

- der veretherten Fettsäurepropoxylate der allgemeinen Formel

$$R\text{-}COO\text{-}X_n\text{-}Y_m\text{-}R',$$

- der Fettsäureethoxylate/propoxylate der allgemeinen Formel

$$R\text{-}COO\text{-}X_n\text{-}Y_m\text{-}H,.$$

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), P olyethylenglycol(17)stearylether (Steareth-17), Polyethylenglycol(18)stearylether (Steareth-18), Polyethylenglycol(19)stearylether (Steareth-19), Polyethylenglycol(20)-stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol-(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),

Polyethylenglycol(13)cetylether (Ceteth-13), Polyethylenglycol(14)cetylether (Ceteth-14), Polyethylenglycol(15) cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(1 9)cetylether (Ceteth-19), Polyethylenglycol(20) cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(1 6)-isocetylether (Isoceteth-16), Polyethylenglycol(17) isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(1 4) oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)-cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23) stearat, Polyethylenglycol(24)stearat, Polyethylenglycol(25)stearat,

Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol-(17)isostearat, Polyethylenglycol(18)isostearat, Polyethylenglycol(19)isostearat, Polyethylenglycol(20)isostearat, Polyethylenglycol(21 )isostearat, Polyethylenglycol(22)isostearat, Polyethylenglycol(23)isostearat, Polyethylenglycol(24)isostearat, Polyethylenglycol (25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol-(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer K ettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Die folgenden Beispiele sollen die Erfindung erläutern, aber nicht einschränken. Die Zahlenangaben beziehen sich auf Gew.-%, sofern nichts Anderes angegeben ist.

| Beispiel 1 O/W-Creme | Gew.-% |
|---|---|
| Glycerylstearatcitrat | 2 |
| Myristylmyristat | 1 |
| Stearylalkohol | 2 |
| Cetylalkohol | 1 |
| Hydrierte Kokosfettglyceride (Hydrogenated Coco Glycerides) | 2 |
| Butylenglycol Dicaprylat/Dicaprat | 1 |
| Ethylhexylkokosfettsäureester | 3 |
| Vaseline | 1 |
| Cyclomethicon | 3 |
| Dicaprylylether | 1 |
| $TiO_2$ | 1 |
| Ethylhexylmethoxycinnamat | 5 |
| Butylmethoxydibenzoylmethan | 1 |
| Ethylhexylsalicylat | 1 |
| Licochalcon A | 0,01 |
| Cholesterin | 0,01 |

(fortgesetzt)

| Beispiel 1 O/W-Creme | Gew.-% |
|---|---|
| Phenoxyethanol | 0,3 |
| p-Hydroxybenzoesäurealkylester (Paraben) | 0,6 |
| Ethanol denaturiert | 3 |
| Polyacrylsäure (Carbomer) | 0,05 |
| Ammoniumpolyacryloyldimethyltaurat | 0,4 |
| Glycerin | 10 |
| wasser- und/oder öllösliche Farbstoffe | 0,05 |
| Füllstoffe/ Additive (Distärke-phosphat, $SiO_2$, BHT, Talkum, Aluminiumstearat) | 0,1 |
| Parfüm | q.s. |
| Wasser | Ad 100 |

| Beispiel 2 O/W-Creme | Gew.-% |
|---|---|
| Glycerylstearat, selbstemulgierend | 5 |
| Stearylalkohol | 1 |
| Sheabutter | 1 |
| $C_{12-15}$ Alkylbenzoat | 3 |
| Caprylsäure/Caprinsäuretriglyceride | 1 |
| Mineralöl | 1 |
| Cyclomethicon | 2 |
| Dicaprylylether | 3 |
| Ethylhexylmethoxycinnamat | 3 |
| Ethylhexyltriazon | 1 |
| Bis-Ethylhexyloxyphenol-methoxyphenyltriazine | 1 |
| Licochalcon A | 0,005 |
| β-Sitosterol | 0,1 |
| Citronensäure, Natriumsalz | 0.1 |
| p-Hydroxybenzoesäurealkylester (Paraben) | 0,3 |
| Hexamidindiisethionat | 0,04 |
| 1,3-Dimethylol-5,5-dimethyl-hydantoin(DMDM Hydantoin) | 0,2 |
| Iodopropynylbutylcarbamat | 0,1 |
| Ethanol denaturiert | 2 |
| Ammoniumacryloyldimethyltaurat/ Vinylpyrrolidoncopolymere | 0,5 |
| Glycerin | 6 |
| Butylenglycol | 3 |
| Füllstoffe/ Additive (Distärke-phosphat, $SiO_2$, BHT, Talkum, Aluminiumstearat) | 1 |
| Parfüm | q.s. |
| Wasser | Ad 100 |

| Beispiel 3 O/W-Creme | Gew.-% |
|---|---|
| Glycerylstearat, selbstemulgierend | 3 |
| PEG-40-Stearat | 1 |
| Cetylalkohol | 3 |
| $C_{12-15}$ Alkylbenzoat | 2 |
| Caprylsäure/Caprinsäuretriglyceride | 2 |
| Octyldodecanol | 1 |
| Vaseline | 1 |
| Cyclomethicon | 4 |
| Dimethicon | 1 |
| Dicaprylylether | 2 |
| $TiO_2$ | 1 |
| Ethylhexylmethoxycinnamat | 5 |
| 2-Hydroxy-4-Methoxy- benzophenon (Oxybenzone) | 2 |
| Ubichinon (Q10) | 0,01 |
| Licochalcon A | 0,05 |
| Cholesterin | 0,5 |
| Tocopherylacetat | 1 |
| Phenoxyethanol | 0,3 |
| p-Hydroxybenzoesäurealkylester (Paraben) | 0,4 |
| Xanthan Gummi | 0,1 |
| Ammoniumacryloyldimethyltaurat/ Vinylpyrrolidoncopolymere | 0,3 |
| Glycerin | 7 |
| Füllstoffe/ Additive (Distärke-phosphat, $SiO_2$, BHT, Talkum, Aluminiumstearat) | 0,2 |
| Parfüm | q.s. |
| Wasser | Ad 100 |

| Beispiel 4 O/W-Creme | Gew.-% |
|---|---|
| Glycerylstearat, selbstemulgierend | 1 |
| Stearinsäure | 4 |
| Behenylalkohol | 1 |
| Cetylalkohol | 3 |
| Butylenglycol Dicaprylat/Dicaprat | 1 |
| Caprylsäure/Caprinsäuretriglyceride | 2 |
| Jojobaöl | 1 |
| Cyclomethicon | 3 |
| Dimethicon | 1 |
| Dicarprylylcarbonat | 3 |
| Ethylhexylcyanodiphenyl-acrylat (Octocrylen) | 5 |

(fortgesetzt)

| Beispiel 4 O/W-Creme | Gew.-% |
|---|---|
| 2-Hydroxy-4-Methoxy- benzophenon (Oxybenzone) | 3 |
| Phenylbenzimidazol sulfonsäure | 2 |
| Licochalcon A | 0,1 |
| β-Sitosterol | 0,001 |
| Phenoxyethanol | 0,2 |
| p-Hydroxybenzoesäurealkylester (Paraben) | 0,3 |
| Diazolidinylharnstoff | 0,2 |
| Ammoniumpolyacryloyldimethyltaurat | 0,3 |
| Glycerin | 6 |
| Butylenglycol | 3 |
| Füllstoffe/ Additive (Distärke-phosphat, $SiO_2$, BHT, Talkum, Aluminiumstearat) | 0,5 |
| Parfüm | q.s. |
| Wasser | Ad 100 |

| Beispiel 5 O/W-Creme | Gew.-% |
|---|---|
| Glycerylstearat, selbstemulgierend | 2 |
| PEG-40-Stearat | 1 |
| Myristylmyristat | 1 |
| Cetearylalkohol | 2 |
| Sheabutter | 2 |
| $C_{12-15}$ Alkylbenzoat | 3 |
| Caprylsäure/Caprinsäuretriglyceride | 2 |
| Ethylhexylkokosfettsäureester | 1 |
| Vaseline | 2 |
| Cyclomethicon | 5 |
| $TiO_2$ | 1 |
| Ethylhexylmethoxycinnamat | 3 |
| Ethylhexylcyanodiphenyl-acrylat (Octocrylen) | 3 |
| 2-Hydroxy-4-Methoxy- benzophenon (Oxybenzone) | 2 |
| Licochalcon A | 0,005 |
| Campesterol | 0,2 |
| Tocopherylacetat | 0,3 |
| Natriumascorbylphosphat | 0,1 |
| Phenoxyethanol | 0,4 |
| p-Hydroxybenzoesäurealkylester (Paraben) | 0,4 |
| Iodopropynylbutylcarbamat | 0,05 |
| Polyacrylsäure (Carbomer) | 0,1 |

(fortgesetzt)

| Beispiel 5 O/W-Creme | Gew.-% |
|---|---|
| Aluminium Stärke Octenylsuccinat | 0,5 |
| Glycerin | 5 |
| Dipropylenglycol | 2 |
| Füllstoffe | 0,05 |
| Parfüm | q.s. |
| Wasser | Ad 100 |

| Beispiel 6 O/W-Creme | Gew.-% |
|---|---|
| Glycerylsterat, selbstemulgierend | 2,5 |
| PEG-40-Stearat | 1 |
| Cetearylalkohol | 2 |
| Hydrierte Kokosfettglyceride (Hydrogenated Coco Glycerides) | 1 |
| Sheabutter | 2 |
| $C_{12-15}$ Alkylbenzoat | 4 |
| Caprylsäure/Caprinsäuretriglyceride | 1 |
| Octyldodecanol | 1 |
| Vaseline | 1 |
| Octamethyltetrasiloxan (Cyclomethicon) | 4 |
| $TiO_2$ | 1 |
| Ethylhexylcyanodiphenyl-acrylat (Octocrylen) | 5 |
| Phenylbenzimidazol-sulfonsäure | 0,5 |
| Ubichinon (Q10) | 0,03 |
| Licochalcon A | 0,01 |
| β-Sitosterol | 0,01 |
| Iminodisuccinat | 0,2 |
| Phenoxyethanol | 0,5 |
| p-Hydroxybenzoesäurealkylester (Paraben) | 0,1 |
| Diazolidinylharnstoff | 0,2 |
| Xanthan Gummi | 0,1 |
| Glycerin | 9 |
| Additive (Distärkephosphat, $SiO_2$, Talkum, BHT, Aluminiumstearat, γ-Tocopherol) | 0.03 |
| Parfüm | q.s. |
| Wasser | Ad 100 |

| Beispiel 7 O/W-Creme | Gew.-% |
|---|---|
| Polyglyceryl-3-Methylglucosedistearat | 3 |
| Sorbitanstearat | 1 |

(fortgesetzt)

| Beispiel 7 O/W-Creme | Gew.-% |
|---|---|
| Behenylalkohol | 2 |
| Cetylalkohol | 1 |
| Butylenglycol Dicaprylat/Dicaprat | 2 |
| Caprylsäure/Caprinsäuretriglyceride | 1 |
| Hydriertes Polydecen | 1 |
| Octamethyltetrasiloxan (Cyclomethicon) | 3 |
| Dicarprylylcarbonat | 2 |
| Ethylhexylmethoxycinnamat | 5 |
| Butylmethoxydibenzoylmethan | 2 |
| Licochalcon A | 0,005 |
| Cholesterin | 0,1 |
| Tocopherylacetat | 0,3 |
| Iminodisuccinat | 0,2 |
| Phenoxyethanol | 0,4 |
| p-Hydroxybenzoesäurealkylester (Paraben) | 0,2 |
| Ethanol denaturiert | 5 |
| Xanthan Gummi | 0,2 |
| Ammoniumacryloyldimethyltaurate/Vinylpyrrolidoncopolymere | 0,3 |
| Glycerin | 5 |
| Butylenglycol | 5 |
| Additive (Distärkephosphat, $SiO_2$, Talkum, BHT, Aluminiumstearat, $\gamma$-Tocopherol) | 0,1 |
| Parfüm | q.s. |
| Wasser | Ad 100 |

| Beispiel 8 O/W-Creme | Gew.-% |
|---|---|
| Polyethylenglycol(21 )stearyl-ether (Steareth-21) | 2 |
| Polyethylenglycol(2)stearyl-ether (Steareth-2) | 1 |
| Cetearylalkohol | 2 |
| Sheabutter | 1 |
| $C_{12-15}$ Alkylbenzoat | 5 |
| Octyldodecanol | 1 |
| Mineralöl | 1 |
| Octamethyltetrasiloxan (Cyclomethicon) | 2 |
| Dicaprylylether | 2 |
| $TiO_2$ | 1 |
| Ethylhexylmethoxycinnamat | 4 |
| Ethylhexyltriazon | 1 |

(fortgesetzt)

| Beispiel 8 O/W-Creme | Gew.-% |
|---|---|
| Ubichinon (Q10) | 0,02 |
| Licochalcon A | 0,002 |
| Stigmasterol | 0.05 |
| Biotin | 0,02 |
| Trinatrium EDTA | 0,2 |
| Phenoxyethanol | 0,5 |
| Hexamidindiisethionat | 0,1 |
| lodopropinylbutylcarbamat | 0,25 |
| Polyacrylsäure (Carbomer) | 0,1 |
| AmmoniumacryloyldimethyltaurateNinylpyrrolidoncopolymere | 0,3 |
| Glycerin | 6 |
| Additive (Distärkephosphat, $SiO_2$, Talkum, BHT, Aluminiumstearat, γ-Tocopherol) | 0,05 |
| Parfüm | q.s. |
| Wasser | Ad 100 |

| Beispiel 9 O/W-Creme | Gew.-% |
|---|---|
| Cetearylglucosid | 2 |
| Myristylmyristat | 1 |
| Stearylalkohol | 4 |
| $C_{12-15}$ Alkylbenzoat | 2 |
| Caprylsäure/Caprinsäuretriglyceride | 3 |
| Hydriertes Polydecen | 1 |
| Dicarprylylcarbonat | 3 |
| Polydecen | 4 |
| Ethylhexylmethoxycinnamat | 3 |
| Ethylhexylcyanodiphenyl-acrylat (Octocrylen) | 3 |
| Butylmethoxydibenzoylmethan | 1 |
| Licochalcon A | 0,01 |
| Cholesterin | 0,001 |
| Tocopherylacetat | 1 |
| Trinatrium EDTA | 0,1 |
| p-Hydroxybenzoesäurealkylester (Paraben) | 0,4 |
| Diazolidinylharnstoff | 0,1 |
| 2-Ethylhexylglycerinether (Octoxyglycerin) | 0,4 |
| Xanthan Gummi | 0,1 |
| Aluminium Stärke Octenylsuccinate | 0,5 |
| Glycerin | 4 |

(fortgesetzt)

| Beispiel 9 O/W-Creme | Gew.-% |
|---|---|
| Butylenglycol | 2 |
| Additive (Distärkephosphat, $SiO_2$, Talkum, BHT, Aluminiumstearat, $\gamma$-Tocopherol) | 3 |
| Parfüm | q.s. |
| Wasser | Ad 100 |

| Beispiel 10 O/W-Creme | Gew.-% |
|---|---|
| Glycerylsterat, selbstemulgierend | 1 |
| Stearinsäure | 2,5 |
| Behenylalkohol | 2 |
| Cetylalkohol | 3 |
| Hydrierte Kokosfettglyceride (Hydrogenated Coco Glycerides) | 1 |
| $C_{12-15}$ Alkylbenzoat | 2 |
| Octyldodecanol | 2 |
| Octamethyltetrasiloxan (Cyclomethicon) | 2 |
| Dimethylpolysiloxan (Dimethicon) | 1 |
| Dicarprylylcarbonat | 4 |
| $TiO_2$ | 1 |
| Ethylhexylmethoxycinnamat | 7 |
| Phenylbenzimidazolsulfonsäure | 1 |
| 2-Hydroxy-4-Methoxy- benzophenon (Oxybenzone) | 2 |
| Licochalcon A | 0,005 |
| $\beta$-Sitosterol | 0,1 |
| Tocopherylacetat | 0,5 |
| Iminodisuccinat | 0,1 |
| Phenoxyethanol | 0,3 |
| p-Hydroxybenzoesäurealkylester (Paraben) | 0,6 |
| Ethanol denaturiert | 3 |
| Polyacrylsäure (Carbomer) | 0,2 |
| Glycerin | 7 |
| wasser- und/oder öllösliche Farbstoffe | 0,1 |
| Additive (Distärkephosphat, $SiO_2$, Talkum, BHT, Aluminiumstearat, $\gamma$-Tocopherol) | 1 |
| Parfüm | q.s. |
| Wasser | Ad 100 |

| **Beispiel 11: W/O-Creme** | |
|---|---|
| Beispiel 11 W/O-Creme | Gew.-% |
| Polyglyceryl-3-Diisostearat | 5,0 |

(fortgesetzt)

| **Beispiel 11: W/O-Creme** | |
|---|---|
| Beispiel 11 W/O-Creme | Gew.-% |
| Polyglyceryl-2-Dipolyhydroxystearat | 2,5 |
| Cetearylalkohol | 2 |
| Cetylalkohol | 2 |
| $C_{12-15}$ Alkylbenzoat | 8 |
| Caprylsäure/Caprinsäuretriglyceride | 6 |
| Octyldodecanol | 5 |
| Octamethyltetrasiloxan (Cyclomethicon) | 2 |
| Milchsäure | 5 |
| Citronensäure, Natriumsalz | 0,5 |
| Butylmethoxydibenzoylmethan | 0,5 |
| Ethylhexyltriazon | 1 |
| Ethylhexylmethoxycinnamat | 5 |
| Licochalcone A | 0,001 |
| Campesterol | 0,01 |
| Retinylpalmitat | 0,05 |
| Phenoxyethanol | 0,1 |
| p-Hydroxybenzoesäurealkyleste (Paraben) | 0,1 |
| Glycerin | 7.5 |
| Füllstoffe (Distärkephosphat, $SiO_2$, Talkum, Aluminiumstearat, BHT) | 0,2 |
| Parfüm | q.s. |
| Wasser | Ad 100 |

| **Beispiel 12: Hydrodispersion/Gelcreme** | Gew.-% |
|---|---|
| Cetylalkohol | 2 |
| Sheabutter | 1 |
| Caprylsäure/Caprinsäuretriglyceride | 2 |
| Octyldodecanol | 1 |
| Octamethyltetrasiloxan (Cyclomethicon) | 5 |
| Dimethylpolysiloxan (Dimethicon) | 1 |
| Polydecen | 2 |
| Ethylhexylmethoxycinnamat | 3 |
| Bis-Ethylhexyloxyphenol-methoxyphenyltriazin | 0,5 |
| Licochalcon A | 0,005 |
| Cholesterin | 0,1 |
| Natriumascorbylphosphat | 0,05 |
| Iminodisuccinat | 0,2 |

(fortgesetzt)

| **Beispiel 12: Hydrodispersion/Gelcreme** | Gew.-% |
|---|---|
| Phenoxyethanol | 0,3 |
| p-Hydroxybenzoesäurealkyleste (Paraben) | 0,4 |
| Vernetztes Alkylacrylat (Alkylacrylate Cosspolymer) | 0,2 |
| Glycerin | 5 |
| Parfüm | q.s. |
| Wasser | Ad 100 |

| Beispiel 13 Pickeringemulsion | Gew.-% |
|---|---|
| Mikrokristallines Wachs | 4,5 |
| Carnaubawachs | 1,5 |
| Candelillawachs | 4,0 |
| Lanolinöl | 4,0 |
| Bis-Diglyceryl Polyacylädipat-2 | 3,5 |
| Simethicon | 1,0 |
| Isopropyl Palmitat | 3,5 |
| Triisostearin | 3,0 |
| Myristyl Lactat | 4,0 |
| Jojobaöl | 2,0 |
| Hydrogeniertes Polydecen | 2,5 |
| Octyldodecanol | 2,5 |
| Licochalcone A | 0,01 |
| Cholesterin | 0,001 |
| Mikronisiertes Titandioxid (Eusolex T 2000) | 2,0 |
| Titandioxid Cl 77891 | 4,0 |
| Eisenoxide Cl 77491, 77492, 77499 | 3,2 |
| D&C Rot 7 | 0,6 |
| Tocopheryl Acetat | 1,0 |
| Xylitol | 2,0 |
| Glycerin | 5,0 |
| Konservierungsmittel, BHT, Parfum, Aroma | q.s. |
| Wasser | 30,0 |
| Ricinusöl | ad 100 |

| Beispiel 14 Pickeringemulsion | Gew.-% |
|---|---|
| Mikrokristallines Wachs | 4 |
| Ozokerit | 0,5 |
| Carnaubawachs | 2,1 |

(fortgesetzt)

| Beispiel 14 Pickeringemulsion | Gew.-% |
|---|---|
| Candelillawachs | 2,0 |
| Bienenwachs | 1,0 |
| Lauryl-Pyrrolidoncarbonsäure | 3,0 |
| Bis-Diglyceryl Polyacyladipat-2 | 5,0 |
| Isopropyl Palmitat | 4,0 |
| Caprylic/Capric Triglycerid | 5,0 |
| Isodecyl Neopentanoat | 2,0 |
| Dicaprylylcarbonat | 2,0 |
| PVP/Hexadecen Copolymer | 0,5 |
| Licochalcone A | 0,05 |
| β-Sitosterol | 0,002 |
| Ethylhexyl Methoxycinnamat | 0,5 |
| Butyl Methoxydibenzoylmethan | 0,2 |
| Mikronisiertes Titandioxid (Eusolex T 2000) | 2,0 |
| Silica Dimethyl Silylat (Aerosil R972) | 0,3 |
| Interferenz Pigmente | 4,0 |
| Titandioxid Cl 77891 | 3,8 |
| Eisenoxide Cl 77491, 77492, 77499 | 2,2 |
| D&C Rot 33 | 0,3 |
| FD&C Gelb 6 | 1,5 |
| Glycerin | 3,0 |
| Konservierungsmittel, BHT, Parfum, Aroma | q.s. |
| Wasser | 40,0 |
| Ricinusöl | ad 100 |

| Beispiel 15 Pickeringemulsion | Gew.-% |
|---|---|
| Mikrokristallines Wachs | 2,6 |
| Ozokerit | 0,5 |
| Carnaubawachs | 1,8 |
| Candelillawachs | 2,0 |
| $C_{24-40}$ Alkyl Stearat | 2,0 |
| Simethicon | 0,5 |
| Myristyl Lactat | 2,0 |
| Caprylsäure/Caprinsäuretriglyceride | 3,0 |
| Pentaerythrityl Tetraisostearat | 2,0 |
| Dicaprylylcarbonat | 2,0 |
| Squalan | 2,0 |

(fortgesetzt)

| Beispiel 15 Pickeringemulsion | Gew.-% |
|---|---|
| Licochalcone A | 0,001 |
| β-Sitosterol | 0,1 |
| Mikronisiertes Titandioxid (Eusolex T 2000) | 3,0 |
| Polymethylsilsesquioxan (Tospearl 2000B) | 1,0 |
| Interferenz Pigmente | 3,0 |
| Titandioxid CI 77891 | 2,0 |
| D&C Rot 7 | 1,9 |
| D&C Rot 6 | 0,4 |
| D&C Rot 33 | 2,2 |
| D&C Rot 34 | 0,3 |
| Tocopheryl Acetat | 1,5 |
| Ubichinon | 0,1 |
| Xylitol | 2,0 |
| Glycerin | 3,0 |
| Konservierungsmittel, BHT, Parfum, Aroma | q.s. |
| Wasser | 50,0 |
| Ricinusöl | 5,0 |
| Octyldodecanol | ad 100 |

| Beispiel 16 Pickeringemulsion | Gew.-% |
|---|---|
| Mikrokristallines Wachs | 1,5 |
| Ozokerit | 0,5 |
| Carnaubawachs | 1,8 |
| Candelillawachs | 6,0 |
| $C_{24-40}$ Alkyl Stearat | 1,5 |
| Lanolinöl | 2,0 |
| Isopropyl Palmitat | 2,0 |
| Myristyl Lactat | 4,0 |
| Jojobaöl | 3,0 |
| Diisostearyl Malat | 1,2 |
| Dicaprylylcarbonat | 3,0 |
| $C_{12-15}$ Alkyl Benzoat | 8,0 |
| Octyldodecanol | 3,0 |
| Licochalcone A | 0,003 |
| Cholesterin | 0,1 |
| Ethylhexyl Methoxycinnamat | 2,0 |
| Bis-Ethylhexyloxyphenol Methoxyphenyl Triazin | 0,25 |

(fortgesetzt)

| Beispiel 16 Pickeringemulsion | Gew.-% |
|---|---|
| Polymethylsilsesquioxan (Tospearl 2000B) | 3,0 |
| Titandioxid Cl 77891 | 3,0 |
| Eisenoxide Cl 77491, 77492, 77499 | 2,2 |
| D&C Rot 7 | 1,3 |
| FD&C Blau 1 | 0,3 |
| D&C Rot 21 | 0,5 |
| D&C Rot 34 | 0,8 |
| Xylitol | 2,0 |
| Glycerin | 5,0 |
| Konservierungsmittel, BHT, Parfum, Aroma | q.s. |
| Wasser . | 20,0 |
| Diisostearyl Fumarat | ad 100 |

| Beispiel 17 **Pflegestift auf W/O-Basis** | Gew.-% |
|---|---|
| Caprylsäure/Caprinsäuretriglyceride | 8 |
| Octyldodecanol | 7 |
| Paraffinöl | 2 |
| Pentaerythrityltetraisostearat | 2 |
| $C_{12-15}$ Alkylbenzoat | 2 |
| Jojobaöl | 2 |
| PEG-45/ Dodecyl Glycol Copolymer | 3 |
| Polyglyceryl-3 Diisostearat | 2,5 |
| Sucrose Distearate | 0,5 |
| Bis-Diglyceryl Polyacyladipat-2 | 9 |
| Cetylpalmitat | 2,5 |
| $C_{16-36}$ Alkyl Stearate | 14 |
| Carnaubawachs | 1,5 |
| Bienenwachs | 0,5 |
| Ethylhexylcyanodiphenylacrylat (Octocrylen) | 2 |
| Licochalcone A | 0,01 |
| Cholesterin | 0,001 |
| Bismuthoxichlorid (BiOCl) | 2 |
| PTFE | 2,5 |
| Perlglanzpigmente | 3 |
| Rokonsal S1 | 0,4 |
| Glycerin | 5 |
| Parfüm, BHT, Neutralisationsmittel, | q.s |

(fortgesetzt)

| Beispiel 17 **Pflegestift auf W/O-Basis** | Gew.-% |
|---|---|
| Wasser | ad 100 |

| **Beispiel 18 Abdeckstift auf W/O-Basis** | **Gew.-%** |
|---|---|
| Caprylsäure/Caprinsäuretriglyceride | 5 |
| Octyldodecanol | 5 |
| Pentaerythrityltetraisostearat | 4 |
| Simethicone | 0,5 |
| PEG-45/ Dodecyl Glycol Copolymer | 3,5 |
| Bis-Diglyceryl Polyacyladipate-2 | 2 |
| $C_{16-36}$ Alkyl Stearate | 1 |
| $C_{20-40}$ Alkyl Stearate | 8 |
| Carnaubawachs | 1,5 |
| PVP / Eicosene Copolymer | 1 |
| Mikronisiertes Titan Dioxid | 2 |
| Octyl Methoxycinnamate | 2 |
| Licochalcone A | 0,02 |
| β-Sitosterol | 0,01 |
| Nylon-12 | 3 |
| Lauroyl Lysine | 0,5 |
| PMMA | 6 |
| Titandioxid mit $Al_2O_3$ beschichtet | 7 |
| Eisenoxide | 4 |
| Ultramarin | 0,5 |
| Germall II | 0,25 |
| Glycerin | 2 |
| Parfüm, BHT, Neutralisationsmittel, | q.s |
| Wasser | ad 100 |

| **Beispiel 19 Foundation-Stift auf W/O-Basis** | Gew.-% |
|---|---|
| Caprylsäure/Caprinsäuretriglyceride | 5 |
| Octyldodecanol | 5 |
| Dicaprylylcarbonat | 3 |
| Dicaprylylether | 2 |
| Simethicone | 0,5 |
| PEG-45/ Dodecyl Glycol Copolymer | 2 |

(fortgesetzt)

| **Beispiel 19 Foundation-Stift auf W/O-Basis** | Gew.-% |
|---|---|
| Polyglyceryl-3 Diisostearate | 1,5 |
| $C_{16-36}$ Alkylstearat | 2 |
| $C_{20-40}$ Alkylstearat | 8 |
| Licochalcone A | 0,002 |
| β-Sitosterol | 0,02 |
| Bismuthoxichlorid (BiOCl) | 3 |
| Polymethylsilsesquioxane (Tospearl) | 0,5 |
| PMMA | 3 |
| Titandioxid mit $Al_2O_3$ beschichtet | 6 |
| Eisenoxide | 4 |
| Ultramarin | 0,6 |
| Glycerin | 10 |
| Parfüm, BHT, Neutralisationsmittel, | q.s |
| Wasser | ad 100 |

| **Beispiel 20 Sonnenschutzstift auf W/O-Basis** | Gew.-% |
|---|---|
| Caprylsäure/Caprinsäuretriglyceride | 8 |
| Octyldodecanol | 8 |
| Pentaerythrityl Tetraisostearate | 8 |
| Jojobaöl | 1 |
| Polyglyceryl-3 Diisostearate | 2 |
| PEG-30 Di-Polyhydroxystearate | 2,5 |
| $C_{16-36}$ Alkyl Stearate | 1 |
| $C_{20-40}$ Alkyl Stearate | 9 |
| PVP / Eicosene Copolymer | 1 |
| Butyl Methoxydibenzoylmethane | 1 |
| Mikronisiertes Titan Dioxid | 4 |
| Ethylhexylcyanodiphenyl-acrylat (Octocrylen) | 3,6 |
| Octyl Methoxycinnamate | 3,6 |
| Licochalcone A | 0,001 |
| β-Sitosterol | 0,02 |
| Bornitrid | 3 |
| Polymethylsilsesquioxane (Tospearl) | 1 |
| Silica LDP | 1 |
| Glydant Plus | 0,3 |
| Glycerin | 5 |

(fortgesetzt)

| **Beispiel 20 Sonnenschutzstift auf W/O-Basis** | Gew.-% |
|---|---|
| Parfüm, BHT, Neutralisationsmittel, | q.s |
| Wasser | ad 100 |

| **Beispiel 21 Blushstift auf W/O-Basis** | **Gew.-%** |
|---|---|
| Caprylsäure/Caprinsäuretriglyceride | 3 |
| Octyldodecanol | 6 |
| Paraffinöl | 1 |
| Isopopyl Palmitate | 2 |
| Jojobaöl | 1 |
| Lanolinöl | 1 |
| Simethicon | 0,5 |
| PEG-45/ Dodecyl Glycol Copolymer | 2 |
| Polyglyceryl-3-Diisostearat | 2,4 |
| Cetyl Palmitate | 1 |
| $C_{20-40}$ Alkylstearat | 8 |
| Carnaubawachs | 2 |
| Candelillawachs | 1 |
| PVP / Eicosene Copolymer | 0,2 |
| Ethylhexylcyanodiphenylacrylat (Octocrylen) | 5 |
| Octyl Methoxycinnamate | 2,5 |
| Licochalcone A | 0,02 |
| Cholesterin | 0,1 |
| Bismuthoxichlorid (BiOCl) | 2 |
| Bornitrid | 1 |
| Titandioxid mit $Al_2O_3$ beschichtet | 2 |
| Eisenoxide | 6 |
| Perlglanzpigmente | 2 |
| Germall II | 0,25 |
| Glycerin | 10 |
| Parfüm, BHT, Neutralisationsmittel, | q.s |
| Wasser | ad 100 |

## Patentansprüche

**1.** Wirkstoffkombinationen aus

(a) einem oder mehreren Vertreter der Gruppe der Phytosterole und/oder Cholesterin und
(b) Licochalcon A oder einem wäßrigen Extrakt aus Radix Glycyrrhizae inflatae, enthaltend Licochalcon A.

**2.** Wirkstoffkombinationen nach Anspruch 1, **dadurch gekennzeichnet, daß** das oder die Phytosterole und Licochalcon A in Verhältnissen von 1000 : 1 bis 1 : 100, bevorzugt 100 : 1 bis 1 : 25, besonders bevorzugt 5 : 1 bis 1 : 5 vorliegen

**3.** Kosmetische oder dermatologische Zubereitungen, enthaltend Wirkstoffkombinationen nach Anspruch 1 oder 2.

**4.** Zubereitungen nach Anspruch 3, enthaltend 0,00001 - 5 Gew.-% e ines oder mehrerer Phytosterole oder Cholesterin, bevorzugt 0,0001 - 2 Gew.-%, insbesondere 0,01 - 1 Gew.-%.

**5.** Zubereitungen nach Anspruch 2 oder 3, enthaltend 0,0001 bis 5 Gew.-%, insbesondere 0,001 bis 1 Gew.-%, ganz besonders 0,005 bis 0,15 Gew.-% Licochalcon A, bezogen auf das Gesamtgewicht der Zubereitung.

**6.** Zubereitungen nach Anspruch 2, 3 oder 4, enthaltend 0,001 bis 20 Gew.-%, insbesondere 0,05 bis 10 Gew.-%, ganz besonders 0,01 bis 5 Gew.-% an einem oder mehreren Polyolen, bezogen auf das Gesamtgewicht der Zubereitung.

**5.** Verwendung von Wirkstoffkombinationen oder Zubereitungen nach einem oder mehreren der vorstehenden Ansprüche zur Prophylaxe und/oder Behandlung von entzündlichen Hautzuständen und/oder zum Hautschutz bei empfindlich determinierter und trockener Haut.

**6.** Verwendung von Wirkstoffkombinationen oder Zubereitungen nach einem oder mehreren der vorstehenden Ansprüche zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut.

**7.** Verwendung von Wirkstoffkombinationen oder Zubereitungen nach einem oder mehreren der vorstehenden Ansprüche zur Behandlung und/oder Prophylaxe von Pigmentierungsstörungen.

**8.** Verwendung von Wirkstoffkombinationen oder Zubereitungen nach einem oder mehreren der vorstehenden Ansprüche zur Steigerung der Ceramidbiosynthese .

**9.** Verwendung von Wirkstoffkombinationen oder Zubereitungen nach einem oder mehreren der vorstehenden Ansprüche zur Stärkung der Barrierefunktion der Haut.

**10.** Verwendung von Wirkstoffkombinationen oder Zubereitungen nach einem oder mehreren der vorstehenden Ansprüche zur Behandlung und/oder Prophylaxe von funktionellen Störungen der Hautanhangsgebilde.

Europäisches Patentamt

# EUROPÄISCHER RECHERCHENBERICHT

Nummer der Anmeldung
EP 04 02 7648

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | DE 196 52 302 C1 (HENKEL KGAA, 40589 DUESSELDORF, DE)<br>26. März 1998 (1998-03-26)<br>* Seite 1, Zeilen 11-13 *<br>----- | 1-10 | A61K31/575<br>A61K31/12<br>A61K35/78<br>A61P17/00 |
| Y | WO 01/00046 A (COGNIS DEUTSCHLAND GMBH; KROPF, CHRISTIAN; FABRY, BERND; BIERMANN, MAN) 4. Januar 2001 (2001-01-04)<br>* Seite 1, Absatz 2 *<br>----- | 1-10 | |
| Y | DE 197 44 703 C1 (HENKEL KGAA, 40589 DUESSELDORF, DE)<br>11. Februar 1999 (1999-02-11)<br>* Anspruch 1 *<br>----- | 1-10 | |
| Y | WO 03/041675 A (SKINLAB GMBH; GRETHER-BECK, SUSANNE)<br>22. Mai 2003 (2003-05-22)<br>* Ansprüche 1,3-8 *<br>* Seite 10, Absatz 2 *<br>----- | 1-10 | RECHERCHIERTE SACHGEBIETE (Int.Cl.7) |
| Y | DATABASE WPI<br>Section Ch, Week 200165<br>Derwent Publications Ltd., London, GB;<br>Class B05, AN 2001-574426<br>XP002320279<br>& JP 2001 163718 A (MARUZEN SEIYAKU KK)<br>19. Juni 2001 (2001-06-19)<br>abstract<br>* Zusammenfassung *<br>-----<br>-/-- | 1-10 | A61K<br>A61P |

Der vorliegende Recherchenbericht wurde für alle Patentansprüche erstellt

| Recherchenort | Abschlußdatum der Recherche | Prüfer |
|---|---|---|
| München | 7. März 2005 | Peris Antoli, B |

1

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 04 02 7648

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| Y | DATABASE WPI<br>Section Ch, Week 198931<br>Derwent Publications Ltd., London, GB;<br>Class D21, AN 1989-223155<br>XP002320280<br>& JP 01 157909 A (MARUZEN KASEI CO LTD)<br>21. Juni 1989 (1989-06-21)<br>abstract<br>* Zusammenfassung *<br>----- | 1-10 | |
| Y | US 6 214 352 B1 (MATSUKAWA SHINYA)<br>10. April 2001 (2001-04-10)<br>abstract<br>----- | 1-10 | |
| | | | RECHERCHIERTE SACHGEBIETE (Int.Cl.7) |

1 Der vorliegende Recherchenbericht wurde für alle Patentansprüche erstellt

| Recherchenort | Abschlußdatum der Recherche | Prüfer |
|---|---|---|
| München | 7. März 2005 | Peris Antoli, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 04 02 7648

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-03-2005

| Im Recherchenbericht angeführtes Patentdokument | Datum der Veröffentlichung | Mitglied(er) der Patentfamilie | Datum der Veröffentlichung |
|---|---|---|---|
| DE 19652302 C1 | 26-03-1998 | KEINE | |
| WO 0100046 A | 04-01-2001 | AU 5405200 A | 31-01-2001 |
| | | CA 2376818 A1 | 04-01-2001 |
| | | WO 0100046 A1 | 04-01-2001 |
| | | EP 1189522 A1 | 27-03-2002 |
| | | JP 2003516115 T | 13-05-2003 |
| | | NO 20016342 A | 21-12-2001 |
| | | US 6352737 B1 | 05-03-2002 |
| DE 19744703 C1 | 11-02-1999 | WO 9918913 A2 | 22-04-1999 |
| WO 03041675 A | 22-05-2003 | DE 10155200 A1 | 28-05-2003 |
| | | WO 03041675 A2 | 22-05-2003 |
| | | EP 1443895 A2 | 11-08-2004 |
| JP 2001163718 A | 19-06-2001 | KEINE | |
| JP 1157909 A | 21-06-1989 | JP 2584423 B2 | 26-02-1997 |
| US 6214352 B1 | 10-04-2001 | KEINE | |

EPO FORM P0461

Für nähere Einzelheiten zu diesem Anhang : siehe Amtsblatt des Europäischen Patentamts, Nr.12/82